# EUROPEAN PATENT APPLICATION

(11) **EP 0 972 523 A2**
(43) Date of publication of application: **19.01.2000**
(21) Application number: 99401265.6
(22) Date of filing: 27.05.1999
(51) Int. Cl.: A61K 39/12, A61K 39/21, A61K 48/00, C12N 15/33, C12N 15/48, C12N 5/10

(54) **Method and compositions for inducing immunity to viruses**

(30) Priority: 29.05.1998 US 87513
(71) Applicant: Japan Health Science Foundation, Tokyo 103-0001 (JP); Ajinomoto Co., Inc., Tokyo 104-8315 (JP); THOMAS JEFFERSON UNIVERSITY, Philadelphia, Pennsylvania 19107-6799 (US)
(72) Inventor: Kaneko, Yutaro, Tokyo 104-8315 (JP); Kozbor, Danuta, Philadelphia, Pennsylvania 19103 (US)
(74) Representative: Des Termes, Monique

(57) **Abstract**

The present invention relates to methods and compositions for inducing cellular immunity against viruses which undergo mutation by introducing a mutant form of an envelope (env) glycoprotein of the virus with an altered or deleted immunodominant epitope. Also disclosed are vaccines and methods of producing the same.

## Description

### BACKGROUND OF THE INVENTION

### Field of the Invention

The present invention relates to methods for inducing immunity to viruses, and in particular to viruses which undergo mutation. More particularly, the invention relates to methods for increasing the response of cytotoxic T lymphocytes (CTL) against the viruses using a construct which expresses a mutant form of the envelope (env) glycoprotein of the virus containing an altered or deleted immunodominant epitope.

### Discussion of the Background

Immunization of HIV-infected individuals with the env glycoprotein is an experimental approach to active immunotherapy of an established retroviral infection. However, successful clinical trials of HIV vaccine therapy have to overcome not only the significant diversity of the env glycoprotein in primary HIV isolates, but also env-CD4 interaction that is critical in AIDS pathogenesis (Siliciano, 1996, *Curr. Top. Microbiol. Immunol. 205:159-79*). This interaction not only provides the molecular basis for the tropism of HIV (Hwang et al, 1991, *Science 253:71-74*), but also initiates several detrimental events that contribute to depletion of CD4⁺ cells and the development of immunodeficiency in HIV-infected individuals.

In HIV-infected individuals, the rate of CD4⁺ T cell loss exceeds the rate at which CD4⁺ cells are produced through thymic differentiation and/or clonal expansion of mature T cells (Siliciano, 1996, *Curr. Top. Microbiol. Immunol. 205:159-79*), suggesting that both direct and indirect mechanisms of cytopathology are responsible for CD4⁺ T cell depletion during progression to AIDS (Oyaizu et al, 1993, *Blood 82:3392-3400*). These involve cell-cell fusion associated with syncytium formation (Sodroski et al, 1986, *Nature 322:470-474),* the destruction by CD16⁺ cytolytic effector cells in the presence of anti-gp120 antibodies (Lyerly et al, 1987, *Proc. Natl. Acad*. *Sci. USA 84:4601-4605*) or by gp120-specific CD4⁺ cells (Stanhope et al, 1993, *Immunol. 150:4672-4686*), random Vβ T cell deletion (Boyer et al, 1993, *Clin. Exp. Immunol. 92:437-441*), and apoptosis induced by cross-linking of bound gp120 to CD4 followed by antigen-driven activation (Banda et al, 1992, J. *Exp. Med*. *176:1099-1106;* Corbeil, J., M. Tremblay, and D. D. Richman, J. *Exp. Med 183:39-48*).

Among the variable regions of gp120, the V3 loop is known as a dominant neutralizing domain of HIV that demonstrates a high degree of variability among different HIV isolates (Hwang et al, 1991, *Science 253:71-74*). The V3 region is also involved in the env-CD4 postreceptor binding steps in virus entry (Freed et al, 1991, J. *Virol. 65:190-194),* and its structure can influence the ability of HIV isolates to respond to the presence of β-chemokine receptors in the target cells (Trkola et al, 1996, *Nature 384:184-187).* The latter interaction plays an important role in cellular events determining both cellular tropism and syncytium forming phenotype of the virus (Lapham et al, 1996, *Science 274:602-604).* In addition, because the majority of neutralizing antibodies (30% - 80%) in HIV-infected patients recognize linear V3 loop sequences (Vogel et al, 1994, J. *Immunol. 153:1895-1904),* these antibodies may largely contribute to ADCC-mediated cytolysis of uninfected CD4⁺ T cells.

The V3 loop of gp120 is a dominant region of the env glycoprotein that induces both humoral and cell-mediated immunity against HIV, but it is also known to undergo frequent mutations (Siliciano, 1996, *Curr. Top. Microbiol. Immunol. 205:159-79;* Hwang et al, 1991, *Science 253:71-74*). The secondary responses induced by the V3 loop mutated epitopes *in vivo* may drive chronic immune activation leading to accelerated exhaustion of its regenerative capacity resulting from high T cell turnover (Wolthers et al, 1996, *Science 274:1543-1547*). Furthermore, the full-length env glycoprotein expressed on the cell surface or released from HIV-infected cells can trigger detrimental events associated with the env-CD4 interactions which are central in AIDS pathogenesis (Siliciano, 1996, *Curr. Top. Microbiol. Immunol. 205:159-79*).

In view of the aforementioned deficiencies attendant with prior art methods of immunizing patients against viruses which undergo frequent mutation, it is clear that there exists a need in the art for a method which redirects immune responses toward conserved epitopes of the envelope glycoproteins of these viruses, thus inducing cellular immunity to multiple strains of the virus, and without inducing detrimental effects associated with the interaction of the envelope glycoprotein and its cellular receptor.

### SUMMARY OF THE INVENTION

Accordingly, one object of the present invention is to provide a method for inducing cellular immunity to a virus by administering to a patient a nucleic acid encoding an envelope glycoprotein of the virus, in an amount sufficient to induce cellular immunity against the virus, wherein the envelope glycoprotein
(a) contains a modified immunodominant epitope; and
(b) induces cellular immunity to a conserved epitope of the envelope glycoprotein.

Another object of this invention is to provide a novel method of inducing cellular immunity against a virus including:
(a) introducing into antigen presenting cells (APCs) a nucleic acid encoding an envelope glycoprotein of the virus, wherein the envelope glycoprotein contains a modified immunodominant epitope; and
(b) administering to a patient in need thereof an amount of the APCs sufficient to induce cellular immunity to conserved epitopes of the virus.

Another object of the invention is to provide a method for preparing a vaccine against a virus including:
(a) introducing into a vector DNA or liposome a nucleic acid encoding an envelope glycoprotein of the virus, wherein the envelope glycoprotein contains a modified immunodominant epitope; and
(b) mixing the vector DNA or liposome with a suitable adjuvant.

Another object of the invention is to provide a method for preparing a vaccine against a virus including:
(a) introducing into antigen presenting cells (APCs) a nucleic acid encoding an envelope glycoprotein of the virus, wherein the envelope glycoprotein contains a modified immunodominant epitope; and
(b) mixing the APCs with a suitable adjuvant.

Yet another object of the invention is to provide a vaccine for inducing cellular immunity against a virus including:
(a) cells expressing on their surfaces an envelope glycoprotein of the virus, wherein the envelope glycoprotein contains a modified immunodominant epitope; and
(b) an adjuvant.

With the foregoing and other objects, advantages and features of the invention that will become hereinafter apparent, the nature of the invention may be more clearly understood by reference to the following detailed description of the preferred embodiments of the invention and to the appended claims.

### BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:

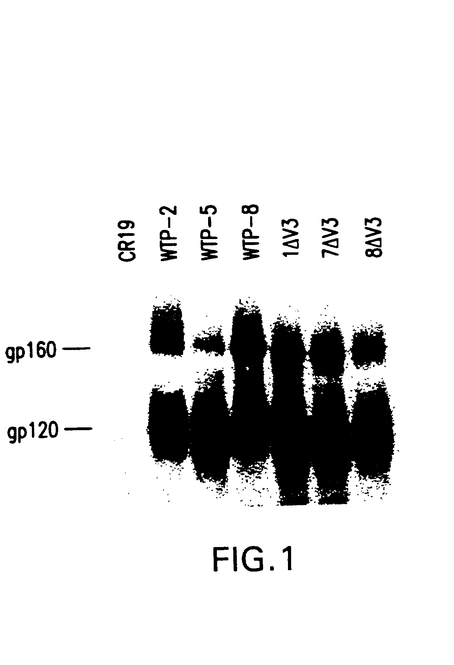

Figure 1 shows the expression of WT and DV3 mutant of HIV env genes. JA2 cells infected with vaccinia viruses expressing the WT and ΔV3 mutant env glycoproteins were immunoprecipitated with serum of an HIV-infected individual and analyzed on SDS-PAGE (8% acrylamide). Cells infected with the CR19 vaccinia virus were used as a negative control.

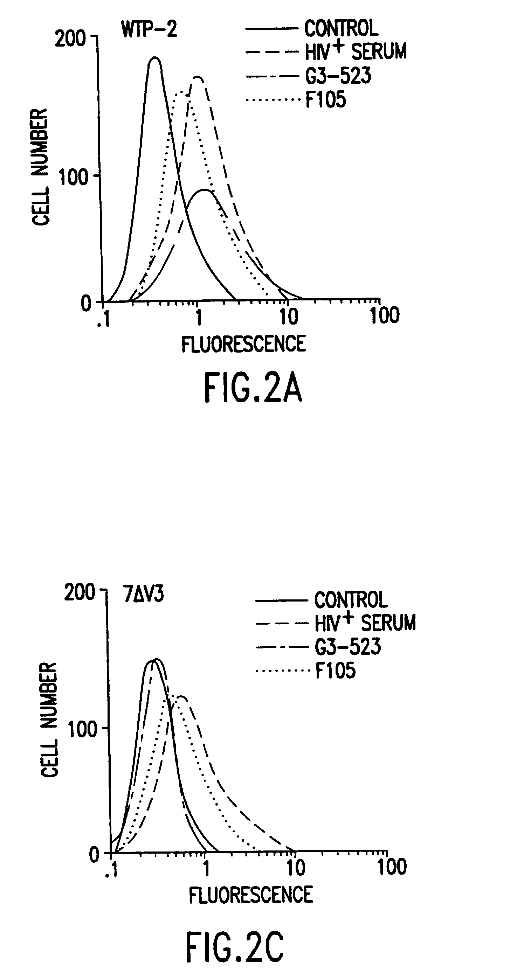

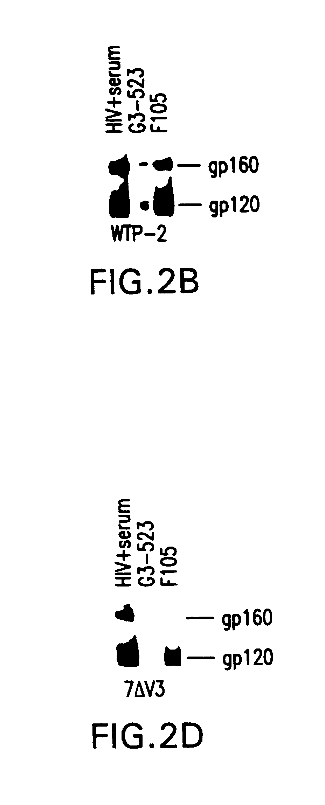

Figure 2 shows immunofluorescence staining and immunoprecipitation of the env glycoprotein expressed by vv-WTP-2 and vv-7ΔV3-infected JA2 cells. For immunofluorescence staining, cells infected with recombinant vaccinia viruses expressing WTP-2 (A) and 7ΔV3 (B) env glycoproteins were incubated with serum of HIV-infected individual (HIV⁺ serum), G3-523 or F105 mAb followed by staining with FITC-conjugated second antibodies, and analyzed by flow cytometry (right panel). Cells infected with the CR19 strain of vaccinia and stained with HIV⁺ serum were used as a negative control. The radiolabeled cell lysates of infected cells were precipitated with the same anti-gp120 antibodies and analyzed by SDS-PAGE (left panel).

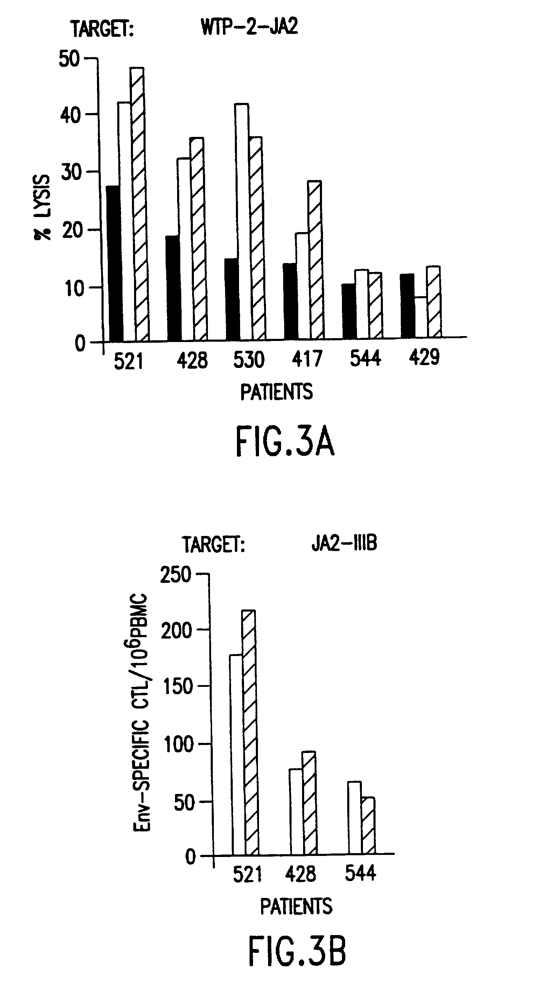

Figure 3 shows induction of env-specific CTL responses in PBMC of HIV-infected individuals by anti-CD3 mAb (closed bars) and autologous LCLs infected with vv-WTP-2 (open bars) or vv-7ΔV3 (hatched bars). The env-specific CTL effectors were tested by ⁵¹Cr-release assay in bulk cultures against vv-WTP-2-infected JA2 cells (E:T ratio of 20:1) using vac-infected cells as a negative control (left panel), and by limiting dilution assay against JA2 cells infected with the HIV-1IIIB isolate (right panel). The frequencies of env-specific CTLp were computed as the difference between CTLp frequencies determined on HIV env versus uninfected control targets, and normalized to the number of CTLp per 10⁶ PBMC.

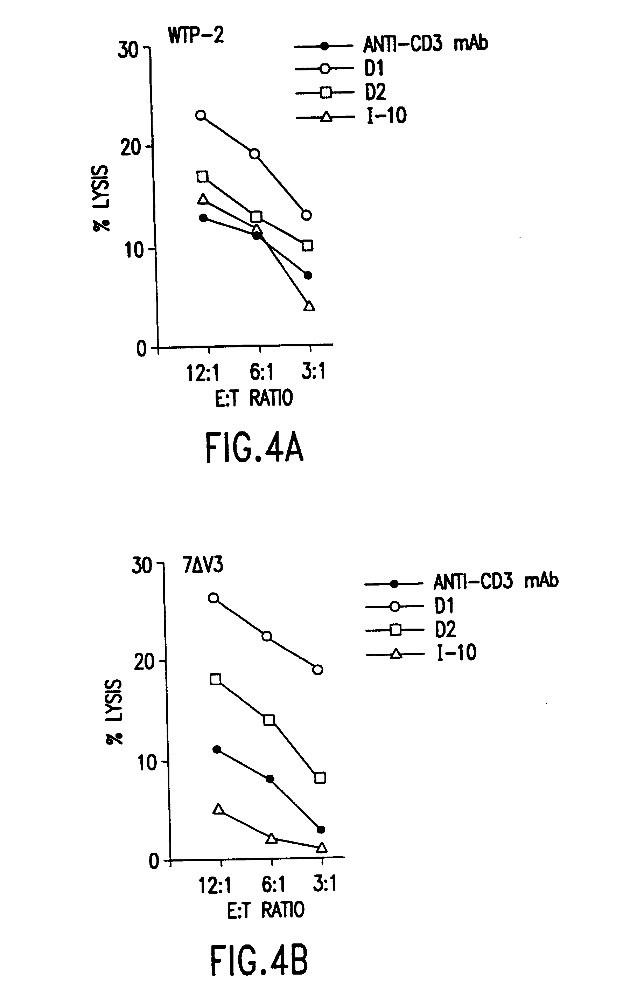

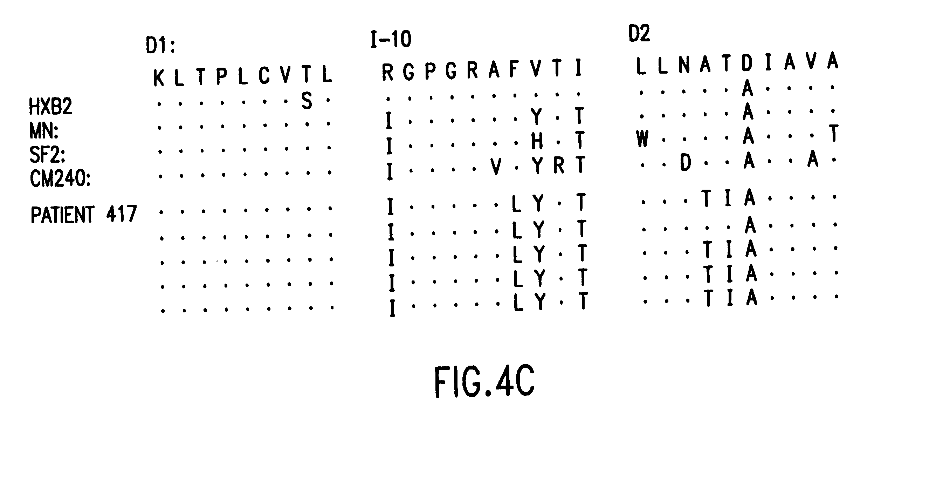

Figure 4 shows CTL activity of env peptide-induced PBMC of HIV-infected patient 417 against JA2 cells (A) and antigenic variation within D1, I-10 and D2 peptides in HIV isolates and natural virus variants of the patient (B). (A) PBMC of patient 417 were stimulated with env-specific peptides at concentration 0.5 µM and tested on day 14 in a ⁵¹Cr-release assay against target cells infected with vv-WTP-2 (left panel) and vv-7ΔV3 (right panel). Cells infected with vac were used as controls, and CTL activity was computed as differences between CTL responses to env versus control target. (B) Antigenic variations in autologous viral sequences in patients 417 within the D1, I-10, and D2 epitopes were determined by RT-PCR amplification of viral env RNA isolated from PBMC. The PCR amplification products were cloned into BlueScript and sequenced with primer. Antigenic variation of HIV isolates were obtained from sequences available from the NCIB Entrez Database using the following accession numbers: K03455 for HXB2; U63632 for JRFL; K02007 for SF2; and U54771 for CM240.

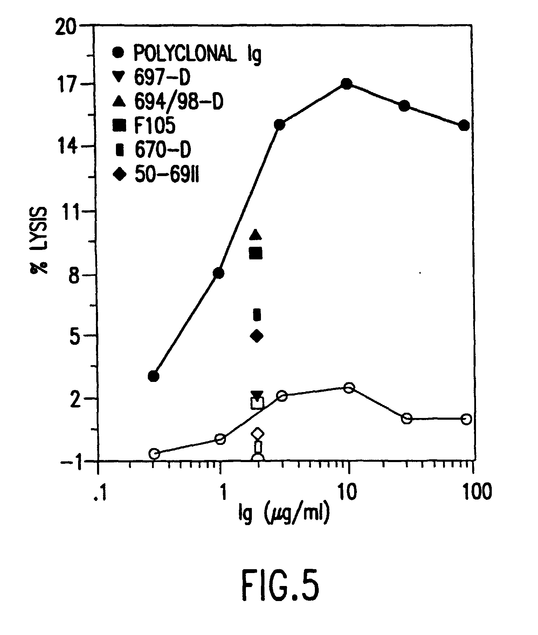

Figure 5 shows ADCC-mediated lysis of allogeneic LCL infected with vv-WTP-2 and vv-ΔV3 in the presence of env-specific antibodies and PBMC of an HIV-seronegative individual. ADCC-specific lysis was calculated by subtracting the background reactivity with the vac-infected target only from the percent cytotoxicity of the vv-ΔV3- or vv-WTP-infected target cells. The E:T ratio was 50:1. All experimental conditions were run in triplicate with standard deviation <5%. Results are representative of three separate experiments.

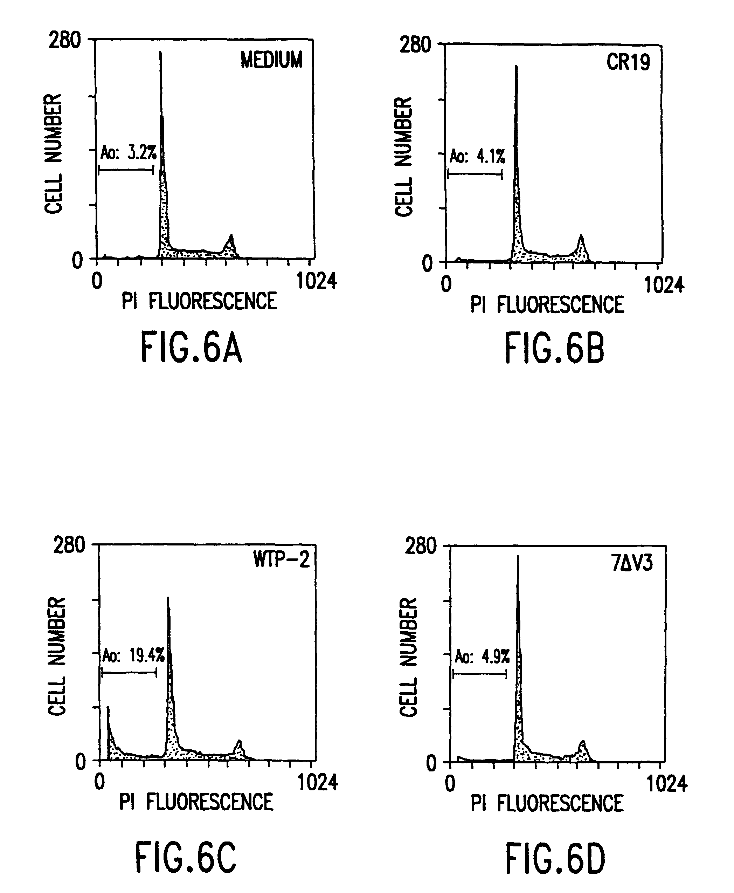

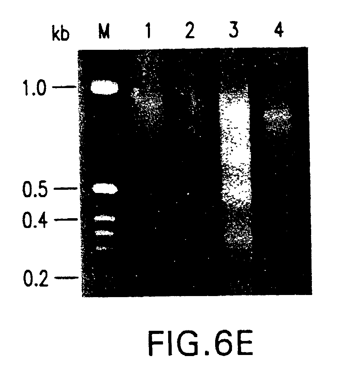

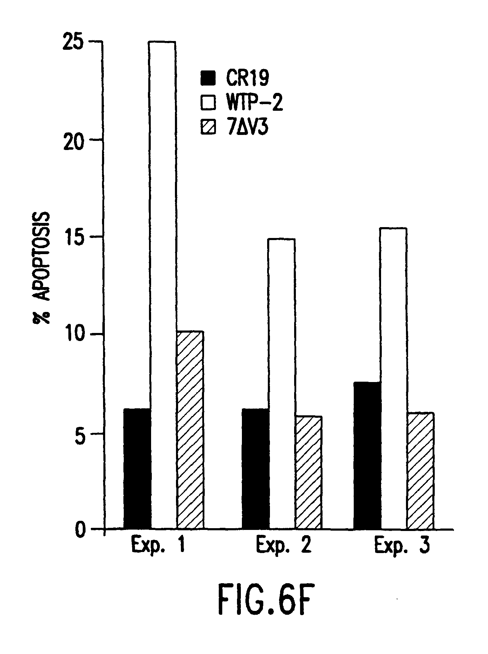

Figure 6 shows detection of env-mediated apoptosis in SupT-1 cells after infection with vaccinia viruses expressing full-length and ΔV3 mutant of env glycoproteins (A and B), and in anti-CD3 mAb-activated IL-2-dependent T cells (C). SupT-1 cells were infected with vac, vv-WTP-2, and vv-7ΔV3 and apoptosis was analyzed in individual cultures 16 hrs after infection by DNA content frequency distributions (A) and DNA gel electrophoresis (B). Uninfected cells were used as a negative control. For quantitation of activation-induced apoptosis, T lymphocytes were incubated with fixed cells expressing env-glycoproteins for 2 hr prior to stimulation with anti-CD3 mAb. Induction of apoptosis was monitored after 72 hr by flow cytometry after staining of nuclei with PI (C).

### DESCRIPTION OF THE PREFERRED EMBODIMENTS

The present inventors have now determined that by modifying an immunodominant epitope of a viral glycoprotein, there is an induction of cell-mediated immune responses and a reduction or elimination of ADCC-mediated lysis of env-expressing cells, syncytium formation and apoptosis. The following definitions further clarify the present invention.

The amino acid residues described herein are preferred to be in the "L" isomeric form. However, residues in the "D" isomeric form can be substituted for any L-amino acid residue, as long as the desired functional property of immunoglobulin-binding is retained by the polypeptide. NH₂ refers to the free amino group present at the amino terminus of a polypeptide. COOH refers to the free carboxy group present at the carboxy terminus of a polypeptide. In keeping with standard polypeptide nomenclature, *J. Biol. Chem.,* **243**:3552-59 (1969), abbreviations for amino acid residues are shown in the following Table of Correspondence:

| TABLE OF CORRESPONDENCE | | |
|---|---|---|
| SYMBOL | AMINO ACID | |
| 1-Letter | 3-Letter | |
| Y | Tyr | tyrosine |
| G | Gly | glycine |
| F | Phe | phenylalanine |
| M | Met | methionine |
| A | Ala | alanine |
| S | Ser | serine |
| I | Ile | isoleucine |
| L | Leu | leucine |
| T | Thr | threonine |
| V | Val | valine |
| P | Pro | proline |
| K | Lys | lysine |
| H | His | histidine |
| Q | Gln | glutamine |
| E | Glu | glutamic acid |
| W | Trp | tryptophan |
| R | Arg | arginine |
| D | Asp | aspartic acid |
| N | Asn | asparagine |
| C | Cys | cysteine |

It should be noted that all amino-acid residue sequences are represented herein by formulae whose left and right orientation is in the conventional direction of amino-terminus to carboxy-terminus. Furthermore, it should be noted that a dash at the beginning or end of an amino acid residue sequence indicates a peptide bond to a further sequence of one or more amino-acid residues. The above Table is presented to correlate the three-letter and one-letter notations which may appear alternately herein.

A "replicon" is any genetic element (e.g., plasmid, chromosome, virus) that functions as an autonomous unit of DNA replication *in vivo;* i.e., capable of replication under its own control.

A "vector" is a replicon, such as plasmid, phage or cosmid, to which another DNA segment may be attached so as to bring about the replication of the attached segment.

A "DNA molecule" refers to the polymeric form of deoxyribonucleotides (adenine, guanine, thymine, or cytosine) in its either single stranded form, or a double-stranded helix. This term refers only to the primary and secondary structure of the molecule, and does not limit it to any particular tertiary forms. Thus, this term includes double-stranded DNA found, *inter alia,* in linear DNA molecules (e.g., restriction fragments), viruses, plasmids, and chromosomes. In discussing the structure of particular double-stranded DNA molecules, sequences may be described herein according to the normal convention of giving only the sequence in the 5' to 3' direction along the nontranscribed strand of DNA (i.e., the strand having a sequence homologous to the mRNA).

An "origin of replication" refers to those DNA sequences that participate in DNA synthesis.

A DNA "coding sequence" is a double-stranded DNA sequence which is transcribed and translated into a polypeptide *in vivo* when placed under the control of appropriate regulatory sequences. The boundaries of the coding sequence are determined by a start codon at the 5' (amino) terminus and a translation stop codon at the 3' (carboxyl) terminus. A coding sequence can include, but is not limited to, prokaryotic sequences, cDNA from eukaryotic mRNA, genomic DNA sequences from eukaryotic (e.g., mammalian) DNA, and even synthetic DNA sequences. A polyadenylation signal and transcription termination sequence will usually be located 3' to the coding sequence.

Transcriptional and translational control sequences are DNA regulatory sequences, such as promoters, enhancers, polyadenylation signals, terminators, and the like, that provide for the expression of a coding sequence in a host cell.

A "promoter sequence" is a DNA regulatory region capable of binding RNA polymerase in a cell and initiating transcription of a downstream (3' direction) coding sequence. For purposes of defining the present invention, the promoter sequence is bounded at its 3' terminus by the transcription initiation site and extends upstream (5' direction) to include the minimum number of bases or elements necessary to initiate transcription at levels detectable above background. Within the promoter sequence will be found a transcription initiation site (conveniently defined by mapping with nuclease S1), as well as protein binding domains (consensus sequences) responsible for the binding of RNA polymerase. Eukaryotic promoters will often, but not always, contain "TATA" boxes and "CAT" boxes. Prokaryotic promoters contain Shine-Dalgarno sequences in addition to the -10 and -35 consensus sequences.

An "expression control sequence" is a DNA sequence that controls and regulates the transcription and translation of another DNA sequence. A coding sequence is "under the control" of transcriptional and translational control sequences in a cell when RNA polymerase transcribes the coding sequence into mRNA, which is then translated into the protein encoded by the coding sequence.

A "signal sequence" can be included before the coding sequence. This sequence encodes a signal peptide, N-terminal to the polypeptide, that communicates to the host cell to direct the polypeptide to the cell surface or secrete the polypeptide into the media, and this signal peptide is clipped off by the host cell before the protein leaves the cell. Signal sequences can be found associated with a variety of proteins native to prokaryotes and eukaryotes.

The term "oligonucleotide," as used herein in referring to the probe of the present invention, is defined as a molecule comprised of two or more ribonucleotides, preferably more than three. Its exact size will depend upon many factors which, in turn, depend upon the ultimate function and use of the oligonucleotide.

The term "primer" as used herein refers to an oligonucleotide, whether occurring naturally as in a purified restriction digest or produced synthetically, which is capable of acting as a point of initiation of synthesis when placed under conditions in which synthesis of a primer extension product, which is complementary to a nucleic acid strand, is induced, i.e., in the presence of nucleotides and an inducing agent such as a DNA polymerase and at a suitable temperature and pH. The primer may be either single-stranded or double-stranded and must be sufficiently long to prime the synthesis of the desired extension product in the presence of the inducing agent. The exact length of the primer will depend upon many factors, including temperature, source of primer and use of the method. For example, for diagnostic applications, depending on the complexity of the target sequence, the oligonucleotide primer typically contains 15-25 or more nucleotides, although it may contain fewer nucleotides.

The primers herein are selected to be "substantially" complementary to different strands of a particular target DNA sequence. This means that the primers must be sufficiently complementary to hybridize with their respective strands. Therefore, the primer sequence need not reflect the exact sequence of the template. For example, a non-complementary nucleotide fragment may be attached to the 5' end of the primer, with the remainder of the primer sequence being complementary to the strand. Alternatively, non-complementary bases or longer sequences can be interspersed into the primer, provided that the primer sequence has sufficient complementarity with the sequence of the strand to hybridize therewith and thereby form the template for the synthesis of the extension product.

As used herein, the terms "restriction endonucleases" and "restriction enzymes" refer to bacterial enzymes, each of which cut double-stranded DNA at or near a specific nucleotide sequence.

A cell has been "transformed" by exogenous or heterologous DNA when such DNA has been introduced inside the cell. The transforming DNA may or may not be integrated (covalently linked) into chromosomal DNA making up the genome of the cell. In prokaryotes, yeast, and mammalian cells for example, the transforming DNA may be maintained on an episomal element such as a plasmid. With respect to eukaryotic cells, a stably transformed cell is one in which the transforming DNA has become integrated into a chromosome so that it is inherited by daughter cells through chromosome replication. This stability is demonstrated by the ability of the eukaryotic cell to establish cell lines or clones comprised of a population of daughter cells containing the transforming DNA. A "clone" is a population of cells derived from a single cell or common ancestor by mitosis. A "cell line" is a clone of a primary cell that is capable of stable growth *in vitro* for many generations.

Two DNA sequences are "substantially homologous" when at least about 75% (preferably at least about 80%, and most preferably at least about 90 or 95%) of the nucleotides match over the defined length of the DNA sequences. Sequences that are substantially homologous can be identified by comparing the sequences using standard software available in sequence data banks, or in a Southern hybridization experiment under, for example, stringent conditions as defined for that particular system. Defining appropriate hybridization conditions is within the skill of the art. See, e.g., Maniatis et al., *supra;* DNA Cloning, Vols. I & II, *supra;* Nucleic Acid Hybridization, *supra,* which is incorporated herein by reference.

It should be appreciated that also within the scope of the present invention are DNA sequences encoding an envelope glycoprotein having the same amino acid sequence as the native envelope glycoprotein, but which are degenerate to the native or wild-type DNA sequences. By "degenerate to" is meant that a different three-letter codon is used to specify a particular amino acid. It is well known in the art that the following codons can be used interchangeably to code for each specific amino acid:

| | |
|---|---|
| Phenylalanine (Phe or F) | UUU or UUC |
| Leucine (Leu or L) | UUA or UUG or CUU or CUC or CUA or CUG |
| Isoleucine (Ile or I) | AUU or AUC or AUA |
| Methionine (Met or M) | AUG |
| Valine (Val or V) | GUU or GUC of GUA or GUG |
| Serine (Ser or S) | UCU or UCC or UCA or UCG or AGU or AGC |
| Proline (Pro or P) | CCU or CCC or CCA or CCG |
| Threonine (Thr or T) | ACU or ACC or ACA or ACG |
| Alanine (Ala or A) | GCU or GCG or GCA or GCG |
| Tyrosine (Tyr or Y) | UAU or UAC |
| Histidine (His or H) | CAU or CAC |
| Glutamine (Gln or Q) | CAA or CAG |
| Asparagine (Asn or N) | AAU or AAC |
| Lysine (Lys or K) | AAA or AAG |
| Aspartic Acid (Asp or D) | GAU or GAC |
| Glutamic Acid (Glu or E) | GAA or GAG |
| Cysteine (Cys or C) | UGU or UGC |
| Arginine (Arg or R) | CGU or CGC or CGA or CGG or AGA or AGG |
| Glycine (Gly or G) | GGU or GGC or GGA or GGG |
| Tryptophan (Trp or W) | UGG |
| Termination codon | UAA (ochre) or UAG (amber) or UGA (opal) |

It should be understood that the codons specified above are for RNA sequences. The corresponding codons for DNA have a T substituted for U.

Mutations can be made in the wild-type sequence such that a particular codon is changed to a codon which codes for a different amino acid. Such a mutation is generally made by making the fewest nucleotide changes possible. A substitution mutation of this sort can be made to change an amino acid in the resulting protein in a non-conservative manner (i.e., by changing the codon from an amino acid belonging to a grouping of amino acids having a particular size or characteristic to an amino acid belonging to another grouping) or in a conservative manner (i.e., by changing the codon from an amino acid belonging to a grouping of amino acids having a particular size or characteristic to an amino acid belonging to the same grouping). Such a conservative change generally leads to less change in the structure and function of the resulting protein. A non-conservative change is more likely to alter the structure, activity or function of the resulting protein. The present invention should be considered to include sequences containing conservative changes which do not significantly alter the activity or binding characteristics of the resulting protein.

The following is one example of various groupings of amino acids:

### Amino acids with nonpolar R groups

Alanine
Valine
Leucine
Isoleucine
Proline
Phenylalanine
Tryptophan
Methionine

### Amino acids with uncharged polar R groups

Glycine
Serine
Threonine
Cysteine
Tyrosine
Asparagine
Glutamine

### Amino acids with charged polar R groups (negatively charged at Ph 6.0)

Aspartic acid
Glutamic acid

### Basic amino acids (positively charged at pH 6.0)

Lysine
Arginine
Histidine (at pH 6.0)

Another grouping may be those amino acids with phenyl groups:
Phenylalanine
Tryptophan
Tyrosine

Another grouping may be according to molecular weight (i.e., size of R groups):

| | |
|---|---|
| Glycine | 75 |
| Alanine | 89 |
| Serine | 105 |
| Proline | 115 |
| Valine | 117 |
| Threonine | 119 |
| Cysteine | 121 |
| Leucine | 131 |
| Isoleucine | 131 |
| Asparagine | 132 |
| Aspartic acid | 133 |
| Glutamine | 146 |
| Lysine | 146 |
| Glutamic acid | 147 |
| Methionine | 149 |
| Histidine (at pH 6.0) | 155 |
| Phenylalanine | 165 |
| Arginine | 174 |
| Tyrosine | 181 |
| Tryptophan | 204 |

Particularly preferred conservative substitutions are:
- Lys for Arg and vice versa such that a positive charge may be maintained;
- Glu for Asp and vice versa such that a negative charge may be maintained;
- Ser for Thr such that a free -OH can be maintained; and
- Gln for Asn such that a free NH₂ can be maintained.

Amino acid substitutions may also be introduced to substitute an amino acid with a particularly preferable property. For example, a Cys may be introduced a potential site for disulfide bridges with another Cys. A His may be introduced as a particularly "catalytic" site (i.e., His can act as an acid or base and is the most common amino acid in biochemical catalysis). Pro may be introduced because of its particularly planar structure, which induces β-turns in the protein's structure.

Two amino acid sequences are "substantially homologous" when at least about 70% of the amino acid residues (preferably at least about 80%, and most preferably at least about 90 or 95%) are identical, or represent conservative substitutions.

A "heterologous" region of the DNA construct is an identifiable segment of DNA within a larger DNA molecule that is not found in association with the larger molecule in nature. Thus, when the heterologous region encodes a mammalian gene, the gene will usually be flanked by DNA that does not flank the mammalian genomic DNA in the genome of the source organism. Another example of a heterologous coding sequence is a construct where the coding sequence itself is not found in nature (e.g., a cDNA where the genomic coding sequence contains introns, or synthetic sequences having codons different than the native gene). Allelic variations or naturally-occurring mutational events do not give rise to a heterologous region of DNA as defined herein.

An "antibody" is any immunoglobulin, including antibodies and fragments thereof, that binds a specific epitope. The term encompasses polyclonal, monoclonal, and chimeric antibodies, the last mentioned described in further detail in U.S. Patent Nos. 4,816,397 and 4,816,567.

An "antibody combining site" is that structural portion of an antibody molecule comprised of heavy and light chain variable and hypervariable regions that specifically binds antigen.

The phrase "antibody molecule" in its various grammatical forms as used herein contemplates both an intact immunoglobulin molecule and an immunologically active portion of an immunoglobulin molecule.

Exemplary antibody molecules are intact immunoglobulin molecules, substantially intact immunoglobulin molecules and those portions of an immunoglobulin molecule that contains the paratope, including those portions known in the art as Fab, Fab', F(ab')₂ and F(v), which portions are preferred for use in the therapeutic methods described herein.

Fab and F(ab')₂ portions of antibody molecules are prepared by the proteolytic reaction of papain and pepsin, respectively, on substantially intact antibody molecules by methods that are well-known. See for example, U.S. Patent No. 4,342,566 to Theofilopolous et al. Fab' antibody molecule portions are also well-known and are produced from F(ab')₂ portions followed by reduction of the disulfide bonds linking the two heavy chain portions as with mercaptoethanol, and followed by alkylation of the resulting protein mercaptan with a reagent such as iodoacetamide. An antibody containing intact antibody molecules is preferred herein.

The phrase "monoclonal antibody" in its various grammatical forms refers to an antibody having only one species of antibody combining site capable of immunoreacting with a particular antigen. A monoclonal antibody thus typically displays a single binding affinity for any antigen with which it immunoreacts. A monoclonal antibody may therefore contain an antibody molecule having a plurality of antibody combining sites, each immunospecific for a different antigen; e.g., a bispecific (chimeric) monoclonal antibody.

The phrase "pharmaceutically acceptable" refers to molecular entities and compositions that are physiologically tolerable and do not typically produce an allergic or similar untoward reaction, such as gastric upset, dizziness and the like, when administered to a human.

The phrase "therapeutically effective amount" is used herein to mean an amount sufficient to prevent, and preferably reduce a feature or pathology such as for example, elevated blood pressure, fever or white cell count as may attend its presence and activity.

A DNA sequence is "operatively linked" to an expression control sequence when the expression control sequence controls and regulates the transcription and translation of that DNA sequence. The term "operatively linked" includes having an appropriate start signal (e.g., ATG) in front of the DNA sequence to be expressed and maintaining the correct reading frame to permit expression of the DNA sequence under the control of the expression control sequence and production of the desired product encoded by the DNA sequence. If a gene that one desires to insert into a recombinant DNA molecule does not contain an appropriate start signal, such a start signal can be inserted in front of the gene.

The term "standard hybridization conditions" refers to salt and temperature conditions substantially equivalent to 5 x SSC and 65 °C for both hybridization and wash. However, one skilled in the art will appreciate that such "standard hybridization conditions" are dependent on particular conditions including the concentration of sodium and magnesium in the buffer, nucleotide sequence length and concentration, percent mismatch, percent formamide, and the like. Also important in the determination of "standard hybridization conditions" is whether the two sequences hybridizing are RNA-RNA, DNA-DNA or RNA-DNA. Such standard hybridization conditions are easily determined by one skilled in the art according to well known formulae, wherein hybridization is typically 10-20°C below the predicted or determined Tₘ with washes of higher stringency, if desired.

As used herein, "cellular immunity" means activation or stimulation of T-lymphocytes against an antigen or antigen-containing cell. Particularly preferred T-cells to be activated are cytotoxic T-cells (i.e., CD8⁺), while particularly preferred T cells to be protected from apoptosis are helper T-cells (i.e., CD4⁺).

By "antigen presenting cell" (APC) is meant any cell which expresses an antigen on its surface, or which engulfs an antigen, processes the antigen, and then inserts at least a portion of the processed antigen into its membrane such that it is exposed extracellularly. Examples of APCs are those of the monocyte/macrophage line and dendritic cells.

By an "immunodominant epitope" is meant a portion of a protein which may be a linear arrangement of amino acids, or may be a non-linear three-dimensional arrangement of amino acids in a protein which is sufficiently exposed as to induce antibodies thereto. Such an epitope may be a "neutralization" or "fusion epitope" to which antibodies which bind and block infection and/or block fusion of cells.

By "a patient in need thereof" is meant a person or animal which is at risk for infection or is already exposed to or infected by a virus.

The virus which contains the envelope glycoprotein is preferably a virus which undergoes antigenic variation such as by antigenic drift (e.g., lentiviruses) or antigenic shift (e.g., influenza). Preferred viruses are retroviruses, and particularly lentiviruses such as human immunodeficiency virus (HIV), equine infectious anemia virus (EIAV), caprine arthritis encephalitis virus (CAEV), simian immunodeficiency virus (SIV), feline immunodeficiency virus (FIV), and visna virus.

As noted above, preferred immunodominant epitopes include the third variable loop (V3) of an envelope glycoprotein, a neutralization epitope, and a fusion epitope. However, it is noted that fusion epitopes may be highly hydrophobic, and thus not well exposed on the glycoprotein. In some cases, cleavage of a precursor glycoprotein, such as the gp160 glycoprotein of HIV into a gp120 and gp41 is required to expose such an epitope.

Any method of altering the immunodominant epitope may be used, including deletion of the epitope, insertion of sequences which disrupt the secondary and tertiary structure of the epitope, and modification by sequences by site-directed mutagenesis. It will be appreciated that alteration by conservative substitution of amino acids will be the least disruptive of the epitope, while substitution of amino acids of different size, structure or charge will have a more significant effect on the structure.

A vaccine effective for treating or preventing a viral infection may be obtained in accordance with the invention by introducing into APCs a nucleic acid encoding a viral envelope glycoprotein containing a modified immunodominant epitope. The nucleic acid is preferably introduced in a vector capable of expressing the modified glycoprotein. However, also contemplated in accordance with the invention are providing the modified glycoprotein directly, or providing a "naked" nucleic acid either alone (in a suitable medium or adjuvant), via a liposome, a gene gun or the like. Also contemplated is providing the nucleic acid using a retroviral, adenoviral or other eukaryotic (e.g., mammalian) expression vector, including but not limited to bovine papilloma virus, vaccinia virus or adeno-associated virus (AAV).

The preparation of therapeutic compositions which contain nucleic acids, polypeptides, analogs or active fragments, or cells containing the foregoing as active ingredients is well understood in the art. Typically, such compositions are prepared as injectables, either as liquid solutions or suspensions, however, solid forms suitable for solution in, or suspension in, liquid prior to injection can also be prepared. The preparation can also be emulsified. The active therapeutic ingredient is often mixed with excipients which are pharmaceutically acceptable and compatible with the active ingredient. Suitable excipients are, for example, water, saline, dextrose, glycerol, ethanol, or the like and combinations thereof. In addition, if desired, the composition can contain minor amounts of auxiliary substances such as wetting or emulsifying agents, pH buffering agents which enhance the effectiveness of the active ingredient.

A nucleic acid, polypeptide, analog or active fragment or cells containing the foregoing can be formulated into the therapeutic composition as neutralized pharmaceutically acceptable salt forms. Pharmaceutically acceptable salts include the acid addition salts (formed with the free amino groups of the polypeptide or antibody molecule) and which are formed with inorganic acids such as, for example, hydrochloric or phosphoric acids, or such organic acids as acetic, oxalic, tartaric, mandelic, and the like. Salts formed from the free carboxyl groups can also be derived from inorganic bases such as, for example, sodium, potassium, ammonium, calcium, or ferric hydroxides, and such organic bases as isopropylamine, trimethylamine, 2-ethylamino ethanol, histidine, procaine, and the like.

The therapeutic nucleic acid-, polypeptide-, analog- or active fragment- or cellular compositions are conventionally administered intravenously, as by injection of a unit dose, for example. The term "unit dose" when used in reference to a therapeutic composition of the present invention refers to physically discrete units suitable as unitary dosage for humans, each unit containing a predetermined quantity of active material calculated to produce the desired therapeutic effect in association with the required diluent; i.e., carrier, or vehicle.

The compositions are administered in a manner compatible with the dosage formulation, and in a therapeutically effective amount. The quantity to be administered depends on the subject to be treated, capacity of the subject's immune system to utilize the active ingredient, and degree of inhibition or neutralization of a particular viral function desired. Precise amounts of active ingredient required to be administered depend on the judgment of the practitioner and are peculiar to each individual. However, suitable dosages may range from about 0.1 to 20, preferably about 0.5 to about 10, and more preferably one to several, milligrams of active ingredient per kilogram body weight of individual per day and depend on the route of administration. Suitable regimes for initial administration and booster shots are also variable, but are typified by an initial administration followed by repeated doses at one or more hour intervals by a subsequent injection or other administration. Alternatively, continuous intravenous infusion sufficient to maintain concentrations of ten nanomolar to ten micromolar in the blood are contemplated.

Having generally described this invention, a further understanding can be obtained by reference to certain specific examples which are provided herein for purposes of illustration only and are not intended to be limiting unless otherwise specified.

### EXAMPLES

### Example 1

### Isolation of cells

PBMC were obtained from heparinized blood of six HIV-infected adults followed in the Clinical Office of the Division of Infectious Diseases at Thomas Jefferson University Hospital, Philadelphia, PA. PBMC of HIV-seronegative donors were obtained from buffy coats (NABI, Miami, FL). The HIV-infected individuals were at early stages of disease with CD4⁺ T cell counts ranging from 300 to 600 cells/mm³. All patients were undergoing antiretroviral therapy with protease inhibitor (crixivan) in combination with dinucleosides; two patients had received zidovudine alone or in combination with lamivudine, and three patients were treated with stavudine alone or in combination with ddI and lamivudine. One patient was on blind study involving stavudine alone vs. crixivan alone vs. combination of both drugs.

All HIV-infected patients expressed HLA-A2 antigen determined by standard serologic techniques performed by the American Red Cross in Philadelphia, PA.

### Example 2

### Preparation of recombinant vaccinia viruses

The HIV-1IIIB isolate was the source of the full-length env gene and the ΔV3 loop mutant cloned in the pSCII-based vector under the control of a synthetic early/late vv promoter (Earl et al, 1990, Removal of cryptic poxvirus transcription termination signals from the human immunodeficiency virus type 1 envelope gene enhances expression and immunogenicity of a recombinant vaccinia virus. *J Virol. 64:2448-2451).* The vv-ΔV3 mutant with the Δ297-329 deletion (15, incorporated by reference herein in its entirety) was constructed by ligation of fragments obtained by PCR amplification from the pSVIII-env plasmid (a gift from Dr. J. Sodroski, Dana-Farber Cancer Institute, Boston, MA). One fragment was generated by PCR with the synthetic oligonucleotide containing the *SaI*I site and the CCACC Kozak's sequence in front of the ATG codon (5'-AGAGTCGACCCACCATGAGAGTGAAGGAGA-3', sense) (SEQ ID NO:1), and the oligonucleotide (5'-ACAGGTACCCCATAATAGACTGTGAC-3', antisense) (SEQ ID NO:2) containing the *Kpn*I side, used for ligation with the second env fragment. The second fragment was derived by *Kpn*I and *Bam*HI digests of the pSVIII-env plasmid, and the third fragment was generated by PCR with the synthetic oligonucleotide containing the *Bam*HI site at its 5' end (5'-AACGGATCCTTAGCACTTATCTGGG-3', sense) (SEQ ID NO:3) and the antisense primer (5'-TTGCGCGGCCGCTTATAGCAAAATCCTTTCC-3') (SEQ ID NO:4) containing the TAA stop codon followed by the *Not*I site. The three fragments were ligated into the *Sal*I and *Not*I sites of the pSC11-based vector (a generous gift of Dr. L. Eisenlohr, Thomas Jefferson University, Philadelphia, PA) to generate plasmid pSC-ΔV3. A similar approach was used to generate plasmid with the WT env gene (pSC-WTP) using recombinant clone pIIIB (Hwang, et al., *Science* 253:71-74) kindly provided by Dr. B. Cullen (Howard Hughes Medical Institute, Duke University Medical Center, Durham, NC). Plasmids pSC-AV3 and pSC-WTP were used to generate vv-ΔV3 and vv-WTP by homologous recombination as described (Earl et al, 1990, *J Virol. 64:2448-2451*).

### Example 3

### Preparation of synthetic peptides

The env peptides D1 (KLTPLCVTL, aa. 121-129) (SEQ ID NO:5); D2 (LLNATAIAV aa. 814-822) (SEQ ID NO:6), and I-10 (RGPGRAFVTI, aa. 318-327) (SEQ ID NO:7)have been previously described (Dupuis et al, *1995, J. Immunol. 155:2232-2239;* Takahashi et al, 1996, *J*. *Exp. Med. 183:879-889).* They were synthesized by standard Fmoc-methodology (Otvos, Jr., et al., *Biochim. Biophys. Acta. 1224:68.),* purified by reverse-phase high performance liquid chromatography and characterized by amino acid analysis and laser-desorption mass spectroscopy at The Wistar Institute, Philadelphia, PA.

### Example 4

### Preparation of target cell lines

Autologous B-LCL were established for use as target cells by incubation of PBMC with supernatant from the EBV-producing marmoset cell line B95.8 (American Type Culture Collection, Rockville MD) as described (Kozbor et al, 1981, *J*. *Immunol. 127:1275-1280*). Jurkat cell line, stably transfected with the gene for HLA-A2, and designated JA2 (Tsomides et al, 1994, *J. Exp. Med. 180:1283-1293*), was obtained from Dr. Linda Sherman (The Scripps Research Institute, La Jolla, CA).

### Example 5

### Cytotoxicity assays

Standard ⁵¹Cr-release assays were performed as described (Luzuriaga et al, 1993, *J. Infect. Dis. 167:1008-1013*). Briefly, target cells (HLA-A2⁺ LCLs or JA2 cells) were infected overnight with 5 MOI of the CR19 vaccinia virus (vac) alone or of vaccinia virus expressing either the WT or the ΔV3 loop env gene products and labeled with Na₂⁵¹CrO₄ (DuPont NEN, Boston, MA). After four washings, 10⁴ target cells were combined with T cell lines established from each patient by *in vitro* stimulation with anti-CD3 n-mAb or autologous LCLs infected with either vv-ΔV3 or vv-WT after inactivation by treatment with 0.5% paraformaldehyde (Klein et al, 1995, *J. Exp. Med. 181:1365*). After 4 hrs, supernatants were harvested and radioactivity was measured in a 1470 Wizard γ-counter (Wallac, Gaithersburg, MD). Spontaneous ⁵¹Cr-release was always <15% of maximum release. Specific lysis was calculated as: 100 x ([experimental release - spontaneous release]/[maximum release - spontaneous release]). Percent HIV-specific lysis was calculated by subtracting the percent specific lysis against vac-infected target cells from percent specific lysis against vv-env-infected target cells. The env-specific responses were considered positive if they exceeded the mean lysis of cells infected with vac alone by 3 SD (Luzuriaga et al, 1993, *J. Infect. Dis. 167:1008-1013;* Klein et al, 1995, *J. Exp. Med. 181:1365*).

### Example 6

### Limiting dilution assay of CTL precursors

Frequencies of env-specific CTLP were determined using limiting dilution analysis (Klein et al, 1995, *J. Exp. Med. 181:1365;* Koup et al, 1991, *J. Exp. Med. 174:1593-1600).* Briefly, PBMC were diluted from 18,000 to 667 cells per well in 24 replicate wells of 96-well microtiter plates along with autologous LCLs infected with vv-ΔV3 or vv-WTP after paraformaldehyde treatment. To each well, 10⁴ fixed stimulator cells and 10⁴ autologous irradiated PBMC (6000 rads) were added. At days 1 and 10, cultures were fed with medium containing IL-2 (50 U/ml), split and tested for cytotoxicity on day 16. ⁵¹Cr-labeled target cells (5 x 10³) were added to each well, and supernatants were harvested after 4 hrs. The target cells were JA2 cells persistently infected with HIV-1IIIB or coated with the env peptide D1 at a concentration of 1 µM. The fraction of nonresponding wells was defined as the number of wells in which ⁵¹Cr-release did not exceed the mean plus 3 SD of the spontaneous release of the 24 control wells. Statistical analysis was performed as described by Strijbosch et al. (Strijbosh et al, 1987, *J. Immunol. Meth. 97:133-140*). CTLp frequencies were expressed as number of CTLp/10⁶ PBMC. HIV-specific CTLP frequencies were computed as differences between CTLp frequencies determined on HIV antigen-expressing versus control targets.

### Example 7

### Antibodies

Monoclonal antibodies (mAb) were comprised of: F105, a human mAb (HmAb) directed against an epitope composed of four discontinuous regions of gp120 that partially overlap the binding site for the CD4 receptor (Thali et al, 1991, *J*. *Virol. 65:6188-6193);* G3-523, mouse mAb (MmAb) directed against an epitope within the V3 region (Moore et al, 1993, *J*. *Virol. 67:4785-4796*); 670-D, HmAb directed against epitopes located within the C-terminus of gp120 (Forthal et al, 1995, *AIDS Res. Hum. Retrovir. 11:1095-1099*); 694/98-D, HmAbs directed against a linear epitope (GRAF) located within the V3 loop (Gorny et al, 1993, *J*. *Immunol. 150:635-643*); 697-D, a HmAb that recognizes epitope within the V2 region of gp120 (Forthal et al, 1995, *AIDS Res. Hum. Retrovir. 11:1095-1099*); and 50-69II, HmAb against epitope within the gp41 region of the env glycoprotein (Forthal et al, 1995). HmAb F105 were provided by Dr. M. Posner (New England Deaconess Hospital, Boston, MA), MmAb G3-523 was provided by Dr. M. Fung (Tanox Biosystems Inc., Houston, TX), and HmAb 697-D, 694/98-D, 670-D, and 50-69II were a generous gift from Drs. S. Zolla-Pazner and M. Gorny (Veterans Affairs Medical Center, New York, NY). Polyclonal Ig from sera of HIV-infected donors was obtained by purification over Protein-G column (Pharmacia Biotech, Piscataway, NJ).

### Example 8

### Immunoprecipitation

Immunoprecipitations of vv-ΔV3- and vv-WTP-infected cells were carried out using Nonidet P-40 (NP-40) buffer (0.5% NP-40, 0.5 M NaCI, 10 mM Tris HCl [pH 7.5]) to lyse cells after overnight labeling with 60 µCi/ml of [³⁵S]cysteine and [³⁵S]methionine (DuPont NEN Company, Boston, MA) in cysteine- and methionine-free DMEM medium. Radiolabeled cell lysates were precipitated with a serum from HIV-infected individual (HIV⁺ serum), G3-523, and F105 mAbs. Briefly, the cell lysates were incubated with anti-gp120 antibodies followed by polyclonal rabbit anti-human Ig (Organon Teknika Corp., West Chester, PA), and immunocomplexes were precipitated with protein A-Sepharose CL4B (Pharmacia Biotech, Piscataway, NJ). Samples electrophoresed on a 8% SDS-polyacrylamide gel were analyzed with a storage phosphor imager (Phospholmager 400S; Molecular Dynamics). The relative amounts of the envelope glycoprotein were determined from the volume of pixel counts in the envelope bands.

### Example 9

### Indirect flow cytometry

LCLs infected for 12 hrs with vv-ΔV3 or vv-WTP-2 were incubated for 30 min with HIV⁺ serum or anti-gp120 mAbs (1 µg/ml) in PBS buffer containing 2% FCS and 0.1% sodium azide (PBS/FCS), washed three times in PBS/FCS, stained with a 1:40 dilution of FITC-conjugated F(ab')₂ fragment of goat anti-human or anti-mouse Ig (Organon Teknica Co., West Chester, PA), and fixed by overnight incubation with 1% paraformaldehyde. Cells were washed in PBS/FCS and analyzed on a Coulter Cytofluorograf System, PROFILE II.

### Example 10

### ADCC

Target cells consisted of JA2 cells infected with vac, vv-ΔV3, or vv-WTP, and ⁵¹Cr-labeled. PBMC from HIV-seronegative donors were used as effector cells (effector to target ratio of 50:1) in the presence of env-specific antibodies. Percent specific lysis was calculated according to the formula [(cpm experimental - cpm spontaneous release)/(cpm maxim= - cpm spontaneous release)] x 100. ADCC-specific lysis was calculated by subtracting the background reactivity with the vac-infected target only from the percent cytotoxicity of the vv-ΔV3- or vv-WTP-infected target cells. All experimental conditions were run in triplicate with standard deviation <5%.

### Example 11

### Syncytium-forming assay and induction of apoptosis

For the syncytium-forming assay, SupT-1 cells were infected with vac, vv-WTP, vv-ΔV3. Control cultures included uninfected cells and cells chronically infected with the HIV-1IIIB isolate plated alone or mixed at 1 : 1 ratio. The number of giant cells in each well was determined under an inverted microscope at different time points after infection.

Induction of apoptosis was analyzed during infection of SupT-1 cells with vac, vv-ΔV3, and vv-WTP env glycoproteins as well as in uninfected T lymphocytes stimulated with anti-CD3 mAb in the presence of cells expressing the env glycoproteins. For activation-induced apoptosis, IL-2-dependent CD4⁺ T cell lines were established by repeated stimulation of CD4-enriched PBL derived from HIV-seronegative donors with anti-CD3 mAb in the presence of irradiated APC. THP-1, a monocytic cell line (American Cell Type Culture Collection, Rockville, MD) was infected with vac, vv-ΔV3, vv-WTP for 12 hr, fixed with 0.5% paraformaldehyde for 30 min on ice, and washed three times with PBS. IL-2-dependent T cell blasts (5x10⁵ cells/ml) were preincubated with vv-env-expressing THP-1 cells (10⁶ cells/ml) for 2 hrs prior to stimulation with 100 ng/ml anti-CD3 mAb for 72 hrs (Tuosto, L. et al., 1995. *Eur. J*. *Immunol. 25:2907-2916.*).

### Example 12

### Detection of apoptosis-associated chromatin degradation and DNA content analysis

The percentage of cells undergoing apoptosis was quantitated by a flow cytometry method for determining fragmented nuclei with propidium iodide (PI) staining and DNA gel electrophoresis. Briefly, cells were fixed with 70% ethanol for 3 hrs at 4°C, centrifuged at 800 g for 5 min and cell pellets were resuspended in PBS containing 0.1 mM EDTA(Na)₂, RNAse A at 50 µg/ml (50U/mg), and PI (50 µg/ml). The cells were then washed twice with PBS before analysis by flow cytometry on a Coulter Cytofluorograf System, PROFILE II. For analysis of the DNA laddering characteristic of apoptotic cell death, low molecular weight DNA fragmentation was determined as previously described (Corbeil, J., et al., 1996, J. *Exp. Med. 183:39-48.*). Briefly, the cell pellet was lysed in 0.5 ml hypotonic buffer, pH 8.0 (5 mM Tris-HCL, 20 mM EDTA, 0.5% Triton X-100), and centrifuged at 27,000 g for 10 min. Fragmented DNA was precipitated overnight with 650 µl isopropanol and 100 µl 5M sodium chloride at -20°C. After centrifugation at 27,000 g for 15 min, the precipitates were dried and resuspended in 10 mM Tris, 1 mM EDTA, pH 7.4, 0.5% SDS, and treated with 8 µl RNase A DNase-free (5 mg/ml) at 37°C for 30 min. Electrophoresis was carried out in a 0.75% agarose gel, and the DNA was visualized under UV light after staining with 5 µg/ml ethidium bromide.

### Example 13

### Reverse transcriptase-PCR and sequence analysis

Natural viral sequences within regions corresponding to the env peptides D1, I-10 and D2 were determined by RT-PCR using cellular RNA isolated from PBMC of HIV-infected patients and two sets of primers. One set of primers amplified a region that spanned an env region from nucleotide 1 to 1095 (in accordance with the HIV-1HXB2 sequence, accession number K03455) corresponding to the env aa 1-365 (5'ACA GAA TTC ATG AGA GTG AAG GAG AAA TAT-3', sense) (SEQ ID NO:8) and (5'-GGT CTA GAC CTG AGG ATT GCT TAA AGA TT-3', antisense) (SEQ ID NO:9), and a second set of primers amplified a region from nucleotide 2248 to 2570, corresponding to aa 749-856 (5'-AAC GGA TCC TTA GCA CTT ATC TGG-3', sense) (SEQ ID NO:10) and (5'-ACG TCG ACC TCG AGT TAT AGC AAA ATC CTT TC-3', antisense) (SEQ ID NO: 11). The obtained PCR fragments were cloned into pBluescript II SK +/- vector and sequenced with Reverse and M13-20 Primers at Nucleic Acid Facility, Kimmel Cancer Institute, Thomas Jefferson University.

### Example 14

### Expression of ΔV3 and WT env glycoprotein in vv-infected cells

To examine structure-function relationships between the HIV env glycoprotein and cellular responses to its mutated gene products, a set of recombinant vaccinia viruses was constructed with complete and V3 loop-deleted env genes. Immunoprecipitation of env gene products from metabolically labeled JA2 cells with serum of an HIV-infected individual revealed less than 20% variation among representative recombinant clones (Fig. 1). For functional studies, two clones designated vv-7ΔV3 and vv-WTP-2 were selected with comparable levels of expression of the full-length and mutated env glycoproteins, respectively.

Immunofluorescence staining and immunoprecipitation of env glycoproteins from vv-infected cells (Fig. 2A and B) revealed that cells expressing the 7ΔV3 mutant did not bind the G3-523 anti-V3 loop mAb. They exhibited lower binding of F105 mAb directed to the CD4-binding site of gp120, consistent with previous reports which demonstrated structural relationship between the V3 loop and the C4 region of gp120 glycoprotein (Wyatt, R., M. et al., *J. Virol. 66:6997-7004.*). Despite the differences in binding to the F105 mAb, the reactivity of the vv-7ΔV3-infected cells with serum of an HIV-infected individual suggests that the folding of the oligomeric gp120 on the surface of infected cells has been preserved in the V3 loop-deleted mutant.

### Example 15

### Induction of env-specific CTL responses by the vv-7ΔV3 env mutant

The env-specific CTL activity was induced in PBMC. of HIV-infected individuals by stimulation with anti-CD3 mAb and autologous LCLs expressing either the wild-type or the V3 loop-deleted env glycoprotein. Because the patients were HLA-A2⁺, JA2 cells infected either with vv-WTP-2 or the HIV-1IIIB isolate were used as targets in the ⁵¹Cr-release assay. All HIV-infected individuals exhibited env-specific CTL activity in anti-CD3 mAb-induced cultures of PBMC with specific lysis that varied between 11% and 26% (E:T ratio of 10:1; Fig. 3, left panel). In 4 out of 6 patients, the stimulation of PBMC with autologous LCLs expressing the env glycoproteins resulted in substantial increases of the cytotoxic response. However, the degree of lytic activity varied in individual cultures. For example, in patients 417, 521, and 428 the CTL activity was higher in cultures induced with cells expressing the 7ΔV3 mutant, whereas in the remaining patients the responses to the full-length env gene products were either higher or comparable to those induced by the 7ΔV3 gene products, and similar to nonspecific responses induced with anti-CD3 mAb. Similar variation of CTT, activity was detected against JA2 cells infected with the HIV-1IIIB isolate using a limiting dilution assay. In 3 HIV-infected individuals, the frequencies of CTLp directed against HIV-1IIIB-infected JA2 cells ranged from 49 to 216 CTL/10⁶ PBMC (Fig. 3, right panel). The highest numbers of CTLp were detected in patients 521 and 428 who also showed increased levels of CTL activities in the bulk cultures of induced PBMC.

The heterogeneity of the CTL responses in the induced cultures suggests that differences in the immunogenicity of the complete and mutated env gene products might influence the repertoire of expanded CTL clones in HIV-infected patients. To explore this issue further, CTL activity was tested against vv-7ΔV3- and vv-WTP-2-infected JA2 cells in cultures induced with synthetic peptides D1 (aa. 120-128), D2 (aa. 814-823) and I-10 (aa. 318-327), representing at least three distinct epitopes of the env glycoprotein. In patient 417, who was capable of eliciting CTL responses to all three peptides, the highest activity against the env was detected in cultures induced with peptide D1, whereas the responses to peptides I-10 and D2 were diminished (Fig. 4A). The molecular basis for the lower responses to peptides I-10 and D2 might be related to the increased divergence within autologous viral sequences in these epitopes exhibited by the prevailing HIV isolate of the patient (Fig. 4B). This is in agreement with previous reports demonstrating sequence variation within the V3 loop and the C-terminal region of env glycoprotein of primary HIV isolates (Hwang et al, 1991, *Science 213:71-74*.,Dupuis, M., S. 1995, *J*. *Immunol. 155:2232-2239.*).

It is also notable that cultures induced with the dominant peptide D1 were more efficient in lysing JA2 cells expressing the 7ΔV3 mutant than those induced with the full-length env gene products, and were associated with the prevalence of CD8⁺ T cells (i.e. >70% of total CD3+ T cells). The higher responses against the mutated env gene products in cultures induced with the D1 peptide is in agreement with the increased frequencies of D1-specific CTLp in PBMC of two HIV-individuals stimulated with vv-7ΔV3-infected LCLs as compared to those induced with cells expressing the WTP-2 env gene products (20 and 55 CTL/10⁶ PBMC vs. 12 and 32 CTLp/10⁶ PBMC, respectively). Results of these experiments suggest that deletion of the V3 region redirects the immune responses to conserved epitopes of the env glycoprotein in some HIV-infected individuals.

### Example 16

### ADCC against 7ΔV3 mutant-infected targets

In a subsequent study, the susceptibility of cells expressing the 7ΔV3 mutant and WTP-2 env glycoproteins to ADCC-mediated lysis was studied using PBMC of HIV-seronegative donors and polyclonal Ig, purified from sera of HIV-infected individuals. Results of experiments with PBMC of 3 HIV-seronegative individuals revealed 15% - 20% of specific lysis against vv-WTP-2-infected autologous LCLs at the E:T ratio of 50:1 (Fig. 5). Among five mAbs specific for epitopes within the V2 and V3 loops (697-D and 694/98-D), the CD4-binding site (F105), the C-terminus (670D), and the gp41 region of gp160 (50-69II), the highest ADCC activities against the WTP-2-infected target were mediated by the 694/98-D and F105 mAbs. Antibodies directed to other regions of gp120 had less effect on the ADCC-mediated lysis. In contrast, the specific lysis detected against vv-7ΔV3-infected target cells in the presence of polyclonal anti-gp120 antibodies or the F105 mAb was less than 4% (Fig. 5). The increased resistance of the vv-7ΔV3-infected cells to the ADCC-mediated lysis might be related to conformational changes in the CD4-binding region induced by deletion of the V3 region, as well as lack of reactivity of this molecule with the anti-V3-specific antibodies present in the majority of HIV-infected individuals.

### Example 17

### Syncytium formation and induction of apoptosis by the 7ΔV3 mutant

The role of the V3 loop in the mechanism of induction of apoptosis was analyzed in CD4⁺ SupT-1 cells infected with vaccinia viruses expressing the complete and mutated env glycoproteins. Examination of SupT-1 cells infected with vv-WTP-2 under a light microscope revealed cell clumping followed by appearance of syncytia 12 hr after infection, and routinely, 80% to 90% cells formed giant cells 24 hr after infection. No syncytia were observed in cultures infected with either vaccinia virus alone or the vv-7ΔV3 mutant.

The apoptotic process associated with syncytium formation was analyzed in individual cultures 16 hr after infection. Analysis of DNA content by staining of nuclei with PI indicated that infection of cells with the full-length env glycoprotein resulted in appearance of ~20% of cells with a sub-G1 DNA content representing apoptotic cells (Fig. 6A), concomitant with decreases in the proportion of cells in the G1 phase of the cell cycle (from 39% to 15%). In contrast, the numbers of apoptotic cells in uninfected cultures and cultures infected with vaccinia virus or the 7ΔV3 mutant were lower than 5%. Apoptosis in Sup-T1 cells expressing the wild-type env glycoproteins was also confirmed by visualization of nucleosome-sized DNA multimers of 180-200 bp to form the characteristic "step-ladder" appearance after size separation on agarose gel (Fig. 6B). Sup-T1 cells infected with vac or expressing the 7ΔV3 mutant failed to induce DNA fragmentation over background levels in isolated low molecular weight DNA.

The induction of apoptosis during infection of SupT-1 cells with vv-WTP-2 is in agreement with previous studies which demonstrated that full-length HIV env glycoproteins are responsible for syncytium formation and apoptosis (Corbeil et al, J. *Exp. Med*. *183:39-48;* Laurent-Crawford et al, 1993, *AIDS Res. and Human Retroviruses 9:761-773*). However, it has been shown that HIV only rarely induces apoptosis in infected cell *in vivo* (Finkel et al, 1995, *Nature Medicine 1:129-134*). To analyze the effect of a deletion of the V3 region on induction of apoptosis in uninfected cells, IL-2-dependent CD4⁺ T lymphocytes were induced through the TCR/CD3 complex after incubation with the full-length and mutated env glycoproteins expressed on the surface of APC. For induction of apoptosis, vv-7ΔV3- and vv-WTP-2-infected THP-1 cells were fixed with paraformaldehyde and incubated with IL-2-dependent T lymphocytes for 2 hrs to allow contact between CD4 expressed on T cells and cell surface-associated gp120 prior to stimulation with anti-CD3 mAb. Induction of apoptosis, monitored after 72 hrs by staining of nuclei with PI and flow cytometry analysis, revealed less than 7% of fragmented DNA in cultures infected with vac and the vv-7ΔV3 mutant. On the other hand, T cells preincubated with the full-length env glycoprotein prior to activation, revealed DNA fragmentation approximately three times that of control levels (Fig. 6C).

Although autologous LCLs expressing comparable levels of the full-length and mutated env glycoproteins were capable of inducing CTL activity in PBMC of HIV-infected individuals, cultures stimulated with the mutated env gene products exhibited increased activity against conserved epitopes of the env glycoprotein. Although not intending to be bound by theory, the mechanisms responsible for the increased immunogenicity of the conserved epitopes in the ΔV3 mutant might be related to lower responses of the HIV-infected individuals to V3 due to increased natural sequence variation within this region. Alternatively, induction of CTL responses with APC expressing the full-length env glycoprotein might be less efficient as compared to those stimulated with the mutated env gene products because binding of the entire gp120 to CD4 on uninfected CD4⁺ T cells may suppress the function of these cells, thus reducing help needed for optimal induction of CTL activity (Giovarelli et al, 1988, *J. Immunol. 141:2831;* Landolfo et al, 1985, *Science 229:176;* Romagnani, 1991, *Immunol. Today 12:256*). In addition, it has been demonstrated that free I-10 peptide corresponding to the epitope located within the V3 loop of the HIV-IIIB isolate can inactivate murine CD8⁺ CTL by a self-veto mechanism which involves simultaneous occupancy of the MHC class I molecule and the TCR on the same CTL (Takahashi et al, 1996, *J. Exp. Med. 183:879-889*). The possible implication of this phenomenon for HIV pathogenesis in humans is that when virus-infected cells are lysed and the digested intracellular proteins released into the environment of T cells, the V3 loop-related self-veto effect may inhibit the induction of CTL responses.

The findings that deletion of the V3 region resulted in ~80% reduction of ADCC might be related to the absence of the V3 loop which serves as a target for anti-V3 loop-specific antibodies abundant in sera of HIV-infected individuals (Vogel et al, 1994, *J.* *Immunol. 153:1895-1904*). Secondly, some conformational changes in other epitopes of gp120 may also contribute to a lower binding of some anti-gp120 antibodies, including those directed to the C4 region of the env glycoprotein. The fact that deletion of the V3 loop resulted in lack of both syncytium formation and induction of apoptosis in CD4⁺ cells is consistent with the notion that these processes are triggered by an interaction between cell membrane expression of mature env glycoproteins and the CD4 receptor (Corbeil et al, 1996, J. *Exp. Med. 183:39-48*; Tuosto et al, 1995, *Eur. J*. *Immunol. 25:2907-2916*). The recently discovered physical association between the CD4-gp120 complex with CXCR-4 and CCR-5 co-receptors on cell membranes (Trkola et al, 1996, *Nature 384:184-187;* Lapham et al, 1996, *Science 274:602-604*) suggests that these interactions may initiate events leading to induction of cell death in a particular subset of CD4⁺ cells. It has been suggested that the V3 loop is involved in these interactions (Feng et al, 1996, *Science 272:872-877*), and that its structure may contain or influence the nature of a complex binding site for the chemokine receptors on gp120 (Trkola et al, 1996, *Nature 384:184-187*). For example, it has been shown that deletion of the V3 loop from both HXB2 gp120 and JR-FL gp120 destroys the CD4-induced epitopes for mAbs 48d and 17b (Wyatt et al, 1992, *J. Virol. 66:6997-7004;* Thali et al, 1991, *J*. *Virol. 65:6188-6193),* which may be relevant to the inability of the ΔV3JR-FL gp120 to interact with CCR-5 (Trkola et al, 1996, *Nature 384:184-187*), and single amino-acid changes in the V3 and C4 regions of HIV-1LAI also have major effects on the structure of these epitopes (Moore et al, 1993, *J*. *Virol. 67:4785-4796*).

Vaccination with the ΔV3 mutant induces immune responses to potentially more conserved regions of the env glycoprotein, which will also reduce the need for immunization with HIV strain-specific env glycoproteins. For induction of cellular responses, the ΔV3 mutant could be injected as a plasmid DNA (Okuda et al, 1995, *AIDS Res. Hum. Retrovir. 11:933-943*) or expressed in autologous cells by intracellular immunization (Wu et al, 1996, *J. Virol. 70:3290-7*) prior to injection into the host. In either case, the cells expressing the mutated env gene products are likely to escape from the ADCC-mediated lysis *in vivo* and may not interfere with functional activities of other CD4⁺ cells. The fact that the ΔV3 mutant interacted with anti-gp120 antibodies of HIV-infected individuals suggests that it is capable of inducing antibody responses *in vivo.*

It should be also noted that although the data showed that the ΔV3 mutant does not induce apoptosis in cells in which it is expressed, it is currently unknown to what extent cells expressing the mutated env glycoprotein are susceptible to apoptosis induced by the full-length env glycoprotein.

Having now fully described the invention, it will be apparent to one of ordinary skill in the art that many changes and modifications can be made thereto without departing from the spirit or scope of the invention as set forth herein.

## Claims

1. A therapeutic composition for inducing cellular immunity against a virus which comprises a nucleic acid encoding an envelope glycoprotein of said virus wherein said envelope glycoprotein
a) contains a modified immunodominant epitope ; and
b) induces cellular immunity to a conserved epitope of said envelope glycoprotein.

2. The therapeutic composition of claim 1, wherein said nucleic acid is introduced into antigen presenting cells (APCs) and said APCs are administered to the patient.

3. The therapeutic composition of claim 1, wherein said virus is a lentivirus.

4. The therapeutic composition of claim 2, wherein said lentivirus is human immunodeficiency virus (HIV).

5. The therapeutic composition of claim 1, wherein said immunodominant epitope is the third variable loop (V3) of said envelope glycoprotein.

6. The therapeutic composition of claim 1, wherein said immunodominant epitope is a neutralization epitope.

7. The therapeutic composition of claim 2, wherein said APCs stimulate peripheral blood mononuclear cells (PBMCs).

8. The therapeutic composition of claim 7, wherein said PBMCs exhibit increased cytotoxic T-lymphocyte (CTL) activity against conserved epitopes of the envelope glycoprotein compared to PBMCs stimulated with APCs encoding a full-length envelope glycoprotein.

9. The therapeutic composition of claim 2, wherein said APCs encoding the modified envelope glycoprotein are resistant to antibody-dependent cell-mediated cytotoxicity (ADCC).

10. The therapeutic composition of claim 2, wherein said APCs encoding the modified envelope glycoprotein do not form syncytia.

11. The therapeutic composition of claim 2, wherein said APCs encoding the modified envelope glycoprotein do not undergo apoptosis.

12. The therapeutic composition of claim 2, wherein said APCs encoding the modified envelope glycoprotein induce cellular immunity to said virus without inducing apoptosis of CD4⁺T cells.

13. The therapeutic composition of claim 1, wherein the immunodominant epitope is deleted.

14. A method for preparing a vaccine against a virus comprising :
(a) introducing into a vector DNA or liposome a nucleic acid encoding an envelope glycoprotein of said virus, wherein said envelope glycoprotein contains a modified immunodominant epitope ; and
(b) mixing said vector DNA or liposome with a suitable adjuvant.

15. The method of claim 14, wherein said nucleic acid is introduced into APCs and said APCs are mixed with the adjuvant.

16. The method of claim 14, wherein said virus is a lentivirus.

17. The method of claim 15, wherein said lentivirus is human immunodeficiency virus (HIV).

18. The method of claim 14, wherein said immunodominant epitope is the third variable loop (V3) of said envelope glycoprotein.

19. A vaccine for inducing cellular immunity against a virus comprising :
(a) cells expressing on their surfaces an envelope glycoprotein of said virus, wherein said envelope glycoprotein contains a modified immunodominant epitope, and
(b) an adjuvant.

20. The vaccine of claim 19, wherein said virus is human immunodeficiency virus (HIV).

21. Use of a nucleic acid encoding an envelope glycoprotein of a virus for the manufacture of medicament for inducing cellular immunity against said virus wherein said envelope glycoprotein
a) contains a modified immunodominant epitope ; and
b) induces cellular immunity to a conserved epitope of said envelope glycoprotein.
